# EUROPEAN PATENT APPLICATION

(11) **EP 3 804 618 A1**
(43) Date of publication of application: **14.04.2021**
(21) Application number: 19201612.9
(22) Date of filing: 07.10.2019
(51) Int. Cl.: A61B 5/1455, A61B 5/00, G01R 33/28

(54) **PHOTOPLETHYSMOGRAPHY APPARATUS FOR A MAGNETIC RESONANCE IMAGING UNIT**

(71) Applicant: Koninklijke Philips N.V., 5656 AG Eindhoven (NL)
(72) Inventor: WEISS, Steffen, 5656 AE Eindhoven (NL)
(74) Representative: Philips Intellectual Property & Standards

(57) **Abstract**

The present invention relates to a photoplethysmography apparatus (10) for a magnetic resonance image acquisition unit. The photoplethysmography apparatus comprises a pair of ear defenders 20, a radiation source 30, and a radiation detector 40. The pair of ear defenders are configured to be worn by a user undergoing a magnetic resonance imaging examination. The radiation source is configured to emit radiation at a location inside an ear defender of the pair of ear defenders and/or at a location at a periphery of the ear defender of the pair of ear defenders such that when the user is wearing the pair of ear defenders radiation emitted from the radiation source radiates skin of the user. The radiation detector is configured to detect radiation reflected and/or scattered from the skin of the user.

## Description

### FIELD OF THE INVENTION

The present invention relates to a photoplethysmography apparatus for a magnetic resonance image acquisition unit, and to a trigger system for a magnetic resonance image acquisition unit.

### BACKGROUND OF THE INVENTION

Many Magnetic Resonance (MR) imaging exams/scans with a MR imaging (MRI) unit involve cardiac triggering or gating, mostly achieved by electrocardiogram (ECG) sensors. Even though ECG-based cardiac triggering is routinely used, it can fail. In some patients it is difficult to measure an ECG signal that allows to determine the time of the R-peak, which is key for cardiac triggering of MR scans. In those patients, a finger-tip-based photoplethysmography (PPG) unit (PPU) is used as a replacement. Because ECG-based triggering does work in most patients, and if it does it has been found to be superior to PPG with respect to R-peak detection in terms of delay and time resolution, in the standard workflow only the ECG method is applied. However, if the ECG method fails, then the patient is commonly pulled out of the MR bore to reposition the ECG-electrodes in order to improve signal quality. If this attempt still fails, a PPU unit is applied and used. This workflow causes considerable staff intervention and prolongs the imaging session significantly. One reason for the prolonged imaging session is that the PPU unit must be applied to the finger-tip. An additional problem arises from the fact that especially for fingers the probability of motion is very high, and such motion causes inaccurate PPG signals. Another problem with this workflow is that in case of a temporary corruption of the ECG signal, there is no surrogate signal that can be used for ECG signal restoration.

There is a need to address these issues.

### SUMMARY OF THE INVENTION

It would be advantageous to have an improved ability to provide for cardiac triggering during MRI. The object of the present invention is solved with the subject matter of the independent claims, wherein further embodiments are incorporated in the dependent claims. It should be noted that the following described aspects and examples of the invention apply also to the photoplethysmography apparatus for a magnetic resonance image acquisition unit, as well as to the trigger system for a magnetic resonance image acquisition unit having such an apparatus.

In a first aspect, there is provided a photoplethysmography apparatus for a magnetic resonance image acquisition unit, comprising:
- a pair of ear defenders;
- a radiation source; and
- a radiation detector.

The pair of ear defenders are configured to be worn by a user undergoing a magnetic resonance imaging examination. The radiation source is configured to emit radiation at a location inside an ear defender of the pair of ear defenders and/or at a location at a periphery of the ear defender of the pair of ear defenders such that when the user is wearing the pair of ear defenders radiation emitted from the radiation source radiates skin of the user. The radiation detector is configured to detect radiation reflected and/or scattered from the skin of the user.

In this way, a PPG monitoring system useable for cardiac triggering will always be available for patients undergoing an MRI scan because the patient will always be required to wear ear defenders. The PPG source/light detection parts will be firmly positioned with respect to the patient, thereby mitigating noise caused by movement of PPG sensors. Also, ECG monitoring can still be used for cardiac triggering, but PPG is also always available. This obviates the need to extract the patient if ECG is found not be appropriate for a patient in order to use a different triggering system. Also, the PPG signal can be used along with the ECG signal to provide more robust cardiac triggering, if for example the ECG signal starts to fail.

The radiation can be directed to the skin such that radiation is not directly reflected without being absorbed/modified by haemoglobin in the blood. Thus, the light source can be placed such that the emission surface or area is touching the skin. Then, in the same way that when a bright torch is held against the skin such as against the palm of the hand, light enters the skin and reflects/scatters/and absorbs in a diffuse reflectance process and exits the skin adjacent to the torch (or light source for the PPG apparatus) and is detected.

In an example, the radiation source is mounted to the ear defender of the pair of ear defenders.

Thus, the source of radiation is an integral part of the ear defenders, such as integrated into one ear muff.

In an example, the radiation source is located externally to the ear defender of the pair of ear defenders. An optical fibre is configured to relay radiation from the radiation source to the location inside the ear defender of the pair of ear defenders and/or to the location at the periphery of the ear defender of the pair of ear defenders.

In other words, a radiation source can be held always outside of the MR fields, whilst a passive optical fibre is used in order to enable light to be emitted at the ear in an MR safe manner. Thus, one end of the optical fibre can be associated with the radiation source that is held away from the MRI unit, with the other end running to the ear defender, in order that radiation from the radiation source can irradiate skin of the patient in a safe manner during an MRI exam.

In an example, the radiation source has at least one band of emission tailored to at least one of an oxy/deoxy-Hb-absorption band.

In an example, the radiation detector is mounted to the ear defender of the pair of ear defenders.

In this manner, reflected/scattered radiation from the skin of the patient can be directly detected, providing for high signal to noise. The circuitry associated with the radiation detector is then compatible with the high fields of the MRI unit, for example have high resistance.

In an example, the radiation detector is located externally to the ear defender of the pair of ear defenders.

In this manner, the radiation detector need not have special circuitry that is compatible with the high fields of the MRI unit. However, there is also a possibility that another camera or detector, that is routinely used in the MRI session, can be used as part of the PPG signal detection.

In an example, an optical fibre is configured to relay reflected and/or scatter radiation from inside the ear defender of the pair of ear defenders and/or from the periphery of the ear defender of the pair of ear defenders to the radiation detector.

To put this another way, a radiation detector can be held always outside of the MR fields, whilst a passive optical fibre is used in order to enable light to be scattered and/or reflected at the ear and be detected in an MR safe manner. Also, signal to noise can be increased.

In an example, the radiation detector is an in-bore camera of the magnetic resonance image acquisition unit.

In this manner, the camera that is used normally for in-bore monitoring can be used for light detection as part of a PPG system thereby reducing the need for extra equipment. Thus, the radiation from the radiation source is directed to the skin at a location, such as at the periphery of the ear muff, and the light scattered and/or reflected is collected. The radiation can be relayed to the radiation detector by an optical fibre or just collected as the reflected/scattered radiation radiates away from the skin of the patient. A part of the ear defender can be partially transparent if required, if the skin of the patient inside the ear defender is radiated, thereby enabling the scattered/reflected radiation to exit the ear defender and be detected. However, if the radiation source is located at the periphery of the ear defender, reflected/scattered radiation radiates away from the patient from a locality around where the skin was radiated. Thus, some radiation radiates away from the patient from a skin locality that is outside of the periphery of the ear defender and can directly radiate towards the radiation detector, and the ear defender need not be partially transparent.

In an example, the radiation detector is configured not to have a sensitivity at a radiation illumination band of one or more ambient light illumination sources associated with the magnetic resonance imaging unit.

In other words, ambient lights in the exam room or on the MRI unit itself emit radiation that is not then detected by the detector because the detector is not sensitive to that radiation thereby mitigating noise disturbances in the PPG signal from the ambient lighting.

In a second aspect, there is provided a trigger system for a magnetic resonance image acquisition unit, comprising:
- a photoplethysmography apparatus according to the first aspect;
- an electrocardiogram (ECG) apparatus; and
- a processing unit.

The photoplethysmography apparatus is configured to provide a first signal indicative of a cardiac cycle of the patient to the processing unit. The ECG apparatus is configured to provide a second signal indicative of the cardiac cycle to the processing unit. The processing unit is configured to determine an MRI trigger signal comprising utilization of the first signal and the second signal.

Thus, the PPG signal can be used to augment and reinforce the ECG signal, and can act as a surrogate signal in case the ECG signal is temporarily corrupted or otherwise not available.

In an example, determination of the MRI trigger signal comprises the processing unit being configured, at specific time points, to replace the second signal with the first signal.

In this manner, if the ECG signal starts to become corrupted or otherwise not suitable for triggering, or disappears to starts to disappear, the processing unit determines that this occurs and replaces the ECG signal with the PPG signal in order that a scan can continue in an uninterrupted way.

In an example, determination of the MRI trigger signal comprises the processing unit augmenting the second signal with the first signal.

In an example, determination of the MRI trigger signal comprises the processing unit determining a delay between a peak in the first signal and an ECG-R peak in the second signal.

In other words, both signals (ECG and PPG) can be utilized to provide a new signal that provides a more robust trigger signal, that functions irrespective of whether the ECG signal starts to fail or whether the PPG signal starts to fail, and when both signals are present a new trigger signal can be provided that is better than either the ECG signal or PPG signal individually.

In an example, determination of the MRI trigger signal comprises the processing unit determining a correlation between the first signal and the second signal. In this manner, the PPG signal can be used to provide a trigger point at exactly the same time point in the cardiac cycle as the ECG signal.

In an example, determination of the MRI trigger signal comprises the processing unit generating an artificial ECG signal comprising utilization of the first signal and the second signal, the generation comprising utilization of a machine learning algorithm.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of a photoplethysmography apparatus for a magnetic resonance image acquisition unit;
Fig. 2 shows a schematic set up of an example of a trigger system for a magnetic resonance image acquisition unit; and
Fig. 3 shows signals from an ECG sensor and an in-ear PPG sensor.

### DETAILED DESCRIPTION OF EMBODIMENTS

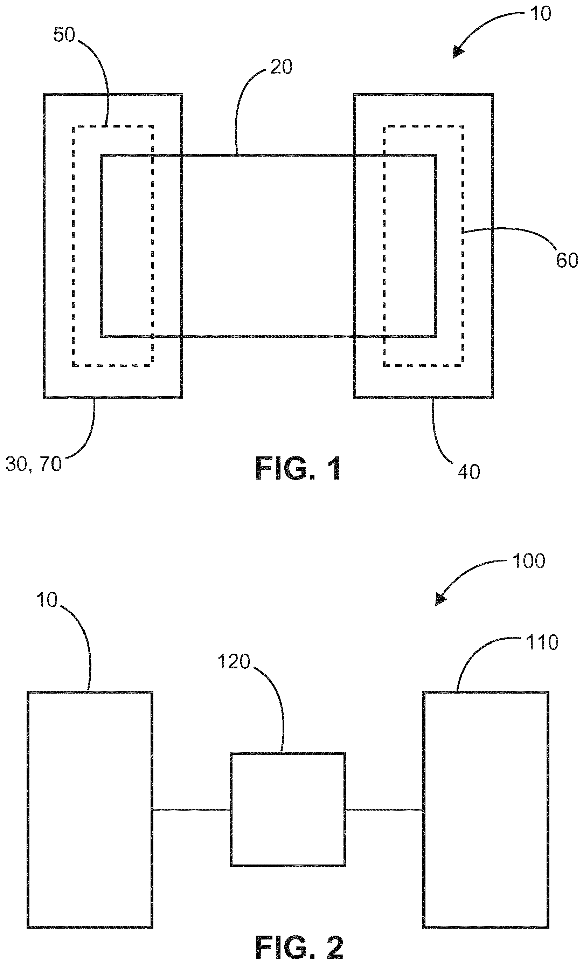

Fig. 1 shows an example of a photoplethysmography (PPG) apparatus 10 for a magnetic resonance image acquisition unit. The PPG apparatus 10 comprises a pair of ear defenders 20, a radiation source 30, and a radiation detector 40. The pair of ear defenders are configured to be worn by a user undergoing a magnetic resonance imaging examination. The radiation source is configured to emit radiation at a location inside an ear defender of the pair of ear defenders and/or at a location at a periphery of the ear defender of the pair of ear defenders such that when the user is wearing the pair of ear defenders radiation emitted from the radiation source radiates skin of the user. The radiation detector is configured to detect radiation reflected and/or scattered from the skin of the user.

In an example, the pair of ear defenders are a pair of ear muffs.

In an example, the pair of ear defenders are a pair of ear plugs or ear buds.

In an example, the radiation source or a second radiation source is configured to emit radiation at a location inside the other ear defender of the pair of ear defenders and/or at a location at a periphery of the other ear defender of the pair of ear defenders such that when the user is wearing the pair of ear defenders radiation emitted from the radiation source radiates skin of the user. In other words, a PPG signal can be acquired from both ears.

According to an example, the radiation source is mounted to the ear defender of the pair of ear defenders.

According to an example, the radiation source is located externally to the ear defender of the pair of ear defenders. An optical fibre 50 can be positioned and used to relay radiation from the radiation source to the location inside the ear defender of the pair of ear defenders and/or to the location at the periphery of the ear defender of the pair of ear defenders.

Thus for example, ear muffs for an MRI exam can be pneumatically activated in order to form an appropriately tight seal around the head of the patient such that the correct level of noise attenuation is provide. The optical fibre can run alongside the air conduit that leads to the ear muffs. However, the optical fibre can run separately, within for example an reinforced conduit that runs to one or both of the ear muffs or to one or both of the ear plugs.

According to an example, the radiation source has at least one band of emission tailored to at least one of an oxy/deoxy-Hb-absorption band.

According to an example, the radiation detector is mounted to the ear defender of the pair of ear defenders.

According to an example, the radiation detector is located externally to the ear defender of the pair of ear defenders.

According to an example, an optical fibre 60 is configured to relay reflected and/or scatter radiation from inside the ear defender of the pair of ear defenders and/or from the periphery of the ear defender of the pair of ear defenders to the radiation detector.

Thus for example, for ear muffs that are pneumatically activated the optical fibre can run alongside the air conduit that leads to the ear muffs. However, the optical fibre can run separately, within for example a reinforced conduit that runs to one or both of the ear muffs or to one or both of the ear plugs.

According to an example, the radiation detector is an in-bore camera 70 of the magnetic resonance image acquisition unit.

According to an example, the radiation detector is configured not to have a sensitivity at a radiation illumination band of one or more ambient light illumination sources associated with the magnetic resonance imaging unit.

Fig. 2 shows an example of a trigger system 100 for a magnetic resonance image acquisition unit. The trigger system comprises a photoplethysmography apparatus 10 as described with respect to Fig. 1. The trigger system also comprises an ECG apparatus 110, and a processing unit 120. The photoplethysmography apparatus is configured to provide a first signal indicative of a cardiac cycle of the patient to the processing unit. The ECG apparatus is configured to provide a second signal indicative of the cardiac cycle to the processing unit. The processing unit is configured to determine an MRI trigger signal comprising utilization of the first signal and the second signal.

In an example, when a PPG signal is acquired from both ears, the processing unit is configured to perform a coincidence analysis for both ears (assuming left-right symmetry), thereby improving the integrity of the overall PPG signal,

According to an example, determination of the MRI trigger signal comprises the processing unit at specific time points replacing the second signal with the first signal.

According to an example, determination of the MRI trigger signal comprises the processing unit augmenting the second signal with the first signal.

According to an example, determination of the MRI trigger signal comprises the processing unit determining a delay between a peak in the first signal and an ECG-R peak in the second signal.

According to an example, determination of the MRI trigger signal comprises the processing unit determining a correlation between the first signal and the second signal.

According to an example, determination of the MRI trigger signal comprises the processing unit generating an artificial ECG signal comprising utilization of the first signal and the second signal, the generation comprising utilization of a machine learning algorithm.

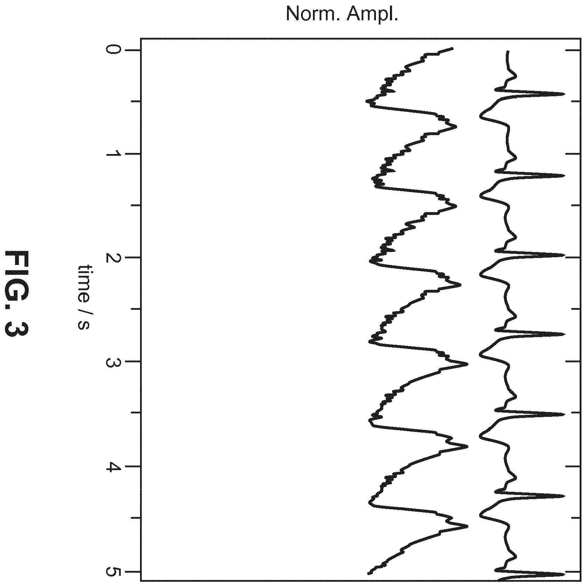

The photoplethysmography apparatus for a magnetic resonance image acquisition unit, and trigger system for a magnetic resonance image acquisition unit having such an apparatus are now described in specific detail, where reference is made to Fig. 3.

As discussed above, ECG monitoring for cardiac triggering in MRI is normally used, but can fail and this requires that the patient be extracted and the ECG system reconfigured and if that does not work the patient is again extracted and another technique such as PPG utilized, which leads to significant delays. This situation is addressed by the developed new technology described here by integrating a PPG unit (PPU) with standard ear muffs, enabling the cardiac signal always to be measured. This PPG signal is superior to that from a standard PPU, such as a finger tip unit, because the sensor always stays firmly attached to the body and is less frequently moved than the finger-tip sensor, because such ear defenders are necessarily securely located with respect to the patient's ears in order to provide the correct level of noise attenuation. Thus by integrating the PPU unit with the ear protection a PPG signal is virtually always available. This is because ear protection of some form is almost always used during the MR exam, and as discussed above ear protection devices such as ear muffs and ear plugs are firmly connected to the body. By integrating the PPG into the ear defenders, motion artefacts are minimized in the PPG signal compared to finger-tip sensors. Such integrated ear-based PPG sensors will always be present and ready to use in any MR exam, and therefore they can be used immediately when the ECG method fails. The decision to use them may be done by the MR system itself or by staff per user input at the console or at the operating panel next to the bore. However, the PPG signal can actually be used to augment the ECG signal to provide a new improved signal for cardiac triggering.

The authors realised after reviewing the papers by Da He and Kaufmann (Da He: "The Ear as a Location for Wearable Vital Signs Monitoring". IEEE EMBS 2010, and Kaufmann S, "In-Ear Pulse Wave Measurements": A Pilot Study. Biomedizinische Technik 2013) that PPG measured at the ear was actually superior to that measured at the finger-tip, and by integrating the PPU into ear defenders an improved cardiac triggering system for MRI could be enabled. Fig. 3 shows an ECG sensor signal (top) and PPG in-ear sensor signal (bottom) used to measure the heart beat (after Kaufmann detailed above) highlighting the efficacy of such a PPG ear based signal.

As the ECG signal is still superior to the PPG signal, it was realised that the ECG signal could be measured as normal, but now to additionally always measure the PPG signal. This provides the possibility to process and improve the ECG signal based on the PPG signal (and indeed vice versa). In a simple embodiment the patient-specific delay of the PPG signal peak relative to the ECG-R-peak is measured as long as both signals are available. However, the correlation between both signals may be measured. This can be used to determine whether R-peaks of the ECG signal have been missed. In case of reliable signal quality of the PPG signal, R-peaks may be mimicked based on the patient-specific delay or based on the entire correlation of the both types of signals. Additionally, it was realised that neuronal networks or machine learning methods in general may be used to create an artificial (improved) ECG-signal based on the (partially disturbed) ECG raw data and the PPG signal. Thus, training ECG signal data and training PPG data can be used to train a machine learning algorithm to in effect provide a new simulated signal that runs whether both the ECG and PPG signal is available, but also runs if either of those signals fail.

### Integration of PPU with ear protection devices

The integration may be done in various ways, including:
Illumination behind earlobe, or behind auricle, reflectance detector on same or transmission detector on other side;
Illumination into ear plug, detector behind auricle or at tragus

A remote camera may be used instead of or in addition to a local optical sensor. This further simplifies the hardware development of the system, as only a suitable illumination needs to be integrated with the ear protection device.

### PPG signal detection and processing may include

Narrow (multi-)band illumination tailored to a strongest response of oxy/deoxy-Hb-absorption change

Light bands in scan room illumination can be avoided to minimise any disturbance

Coincidence analysis for both ears (assuming left-right symmetry)

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A photoplethysmography apparatus (10) for a magnetic resonance image acquisition unit, comprising:
- a pair of ear defenders (20);
- a radiation source (30); and
- a radiation detector (40);
wherein the pair of ear defenders are configured to be worn by a user undergoing a magnetic resonance imaging examination;
wherein the radiation source is configured to emit radiation at a location inside an ear defender of the pair of ear defenders and/or at a location at a periphery of the ear defender of the pair of ear defenders such that when the user is wearing the pair of ear defenders radiation emitted from the radiation source radiates skin of the user;
wherein, the radiation detector is configured to detect radiation reflected and/or scattered from the skin of the user.

2. Photoplethysmography apparatus according to claim 1, wherein the radiation source is mounted to the ear defender of the pair of ear defenders.

3. Photoplethysmography apparatus according to claim 1, wherein the radiation source is located externally to the ear defender of the pair of ear defenders, and wherein an optical fibre (50) is configured to relay radiation from the radiation source to the location inside the ear defender of the pair of ear defenders and/or to the location at the periphery of the ear defender of the pair of ear defenders.

4. Photoplethysmography apparatus according to any of claims 1-3, wherein the radiation source has at least one band of emission tailored to at least one of an oxy/deoxy-Hb-absorption band.

5. Photoplethysmography apparatus according to any of claims 1-4, wherein the radiation detector is mounted to the ear defender of the pair of ear defenders.

6. Photoplethysmography apparatus according to any of claims 1-4, wherein the radiation detector is located externally to the ear defender of the pair of ear defenders.

7. Photoplethysmography apparatus according to claim 6, wherein an optical fibre (60) is configured to relay reflected and/or scatter radiation from inside the ear defender of the pair of ear defenders and/or from the periphery of the ear defender of the pair of ear defenders to the radiation detector.

8. Photoplethysmography apparatus according to any of claims 6-7, wherein the radiation detector is an in-bore camera (70) of the magnetic resonance image acquisition unit.

9. Photoplethysmography apparatus according to any of claims 1-8, wherein radiation detector is configured not to have a sensitivity at a radiation illumination band of one or more ambient light illumination sources associated with the magnetic resonance imaging unit.

10. A trigger system (100) for a magnetic resonance image acquisition unit, comprising:
- a photoplethysmography apparatus (10) according to any of claims 1-9;
- an ECG apparatus (110);
- a processing unit (120);
wherein, the photoplethysmography apparatus is configured to provide a first signal indicative of a cardiac cycle of the patient to the processing unit;
wherein, the ECG apparatus is configured to provide a second signal indicative of the cardiac cycle to the processing unit; and
wherein, the processing unit is configured to determine an MRI trigger signal comprising utilization of the first signal and the second signal.

11. Trigger system according to claim 10, wherein determination of the MRI trigger signal comprises the processing unit at specific time points to replacing the second signal with the first signal.

12. Trigger system according to any of claims 10-11, wherein determination of the MRI trigger signal comprises the processing unit augmenting the second signal with the first signal.

13. Trigger system according to any of claims 10-12, wherein determination of the MRI trigger signal comprises the processing unit determining a delay between a peak in the first signal and an ECG-R peak in the second signal.

14. Trigger system according to any of claims 10-13, wherein determination of the MRI trigger signal comprises the processing unit determining a correlation between the first signal and the second signal.

15. Trigger system according to any of claims 10-14, wherein determination of the MRI trigger signal comprises the processing unit generating an artificial ECG signal comprising utilization of the first signal and the second signal, the generation comprising utilization of a machine learning algorithm.
